# EUROPEAN PATENT SPECIFICATION

(11) **EP 1 534 326 B1**
(45) Date of publication and mention of the grant of the patent: **04.07.2007**
(21) Application number: 03791150.0
(22) Date of filing: 01.09.2003
(51) Int. Cl.: A61K 39/095, A61P 37/04

(54) **IMPROVED BACTERIAL OUTER MEMBRANE VESICLES**
MEMBRANVESIKEL (OMV) MIT VERBESSERTEN EIGENSCHAFTEN
AMELIORATIONS APPORTEES A DES VESICULES DE MEMBRANE EXTERNE BACTERIENNES

(30) Priority: 30.08.2002 GB 0220194
(43) Date of publication of application: 01.06.2005
(73) Proprietor: Novartis Vaccines and Diagnostics S.r.l., 53100 Siena SI (IT)
(72) Inventor: PIZZA, Mariagrazia, I-53100 Siena (IT); SERRUTO, Davide, I-53100 Siena (IT); RAPPUOLI, Rini, I-53100 Siena (IT)
(74) Representative: Marshall, Cameron John
(86) International application number: PCT/IB2003/004293
(87) International publication number: WO 2004/019977

(56) References cited:
- WO-A-01/91788
- WO-A-02/062378
- US-A1- 2002 110 569

## Description

### TECHNICAL FIELD

This invention is in the field of vesicle preparation for immunisation purposes.

### BACKGROUND ART

One of the various approaches to immunising against *N.meningitidis* infection is to use outer membrane vesicles (OMVs). An efficacious OMV vaccine against serogroup B has been produced by the Norwegian National Institute of Public Health [*e.g.* ref. 1 but, although this vaccine is safe and prevents MenB disease, its efficacy is limited to the strain used to make the vaccine.

The 'RIVM' vaccine is based on vesicles containing six different PorA subtypes and has been shown to be immunogenic in children in phase Il clinical trials [2].

Reference 3 discloses a vaccine against different pathogenic serotypes of serogroup B meningococcus based on OMVs which retain a protein complex of 65-kDa. Reference 4 discloses a vaccine comprising OMVs from genetically-engineered meningococcal strains, with the OMVs comprising: at least one Class 1 outer-membrane protein (OMP) but not comprising a Class 2/3 OMP. Reference 5 discloses OMVs comprising OMPs which have mutations in their surface loops and OMVs comprising derivatives of meningococcal lipopolysaccharide (LPS).

Reference 6 discloses compositions comprising OMVs supplemented with transferrin binding proteins (*e.g.* TbpA and TbpB) and/or Cu,Zn-superoxide dismutase. Reference 7 discloses compositions comprising OMVs supplemented by various proteins. Reference 8 discloses preparations of membrane vesicles obtained from *N. meningitidis* with a modified *fur* gene.

As well as serogroup B *N.meningitidis,* vesicles have been prepared for other bacteria. Reference 9 discloses a process for preparing OMV-based vaccines for serogroup A meningococcus. References 10 and 11 disclose vesicles from *N.gonorrhoeae.* Reference 12 discloses vesicle preparations from *N.lactamica.* Vesicles have also been prepared from *Moraxella catarrhalis* [13,14], *Shigella flexneri* [15,16], *Pseudomonas aeruginosa* [15,16], *Porphyrnmoncts gingivalis [17], Treponema pallidum* [18], *Flaemophilus influenzae* [19 & 20] and *Helicobacter pylori* [21].

One drawback with bacterial vesicle preparations is that important protective antigens are not present. To retain antigens such as NspA in OMV preparations, reference 20 teaches that *nspA* expression should be up-regulated with concomitant *porA* and *cps* knockout. It is an object of the invention to provide further and improved vesicle preparations, together with processes for their manufacture. In particular, it is an object of the invention to provide vesicles which retain important bacterial immunogenic components from *N.meningitidis.*

### DISCLOSURE OF THE INVENTION

Prior art methods of meningococcal OMV preparation involve the use of detergent during disruption of the bacterial membrane [*e.g.* see ref. 22, where a deoxycholate detergent is used]. The invention is based on the surprising discovery that membrane disruption substantially in the absence of detergent results in OMVs which retain important bacterial immunogenic components, particularly (i) the protective NspA surface protein, (ii) protein '287' and (iii) protein '741'.

Therefore the invention provides a process for the manufacture of an outer membrane vesicle preparation from a bacterium, wherein the bacterial membrane is disrupted substantially in the absence of detergent. OMV preparations obtainable by processes of the invention are also provided.

For obtaining NspA^{+ve} vesicles, the process of the invention is much simpler than performing multiple genetic manipulations as described in reference 20.

The process of the invention will typically involve the following basic steps: (a) treating bacterial cells in the substantial absence of detergent; (b) centrifuging the composition from step (a) to separate the outer membrane vesicles from treated cells and cell debris, and collecting the supernatant; (c) performing a high speed centrifugation of the supernatant from step (b) and collecting the outer membrane vesicles in a pellet; (d) re-dispersing the pellet from step (c) in a buffer; (e) performing a second high speed centrifugation in accordance with step (c), collecting the outer membrane vesicles in a pellet; (f) re-dispersing the pellet from step (e) in an aqueous medium.

The process may also comprise the following steps: (g) performing sterile filtration through at least two filters of decreasing pore size of the re-dispersed composition from step (f); and (h) optionally including the composition from step (g) in a pharmaceutically acceptable carrier and/or adjuvant composition.

Step (a) gives rise to vesicles of the bacterial outer membrane, and the vesicles generally comprise outer membrane components in substantially their native form. Advantageously, membrane components NspA, '287' and '741' are preserved.

Step (b) will typically involve centrifugation at around 5000 - 10000 g for up to 1 hour.

Steps (c) and (e) will typically involve centrifugation at around 35000 - 100000 g for up to 2 hours.

Centrifugation steps are preferably performed at between 2°C and 8°C.

Any suitable buffer can be used in step (d) *e.g*. Tris buffer, phosphate buffer, histidine buffer, *etc.* Step (f) may also involve the use of a buffer, which may be the same buffer as used in step (d), or may simply involve the use of water (*e.g.* water for injection).

Step (g) preferably ends with a filter of pore-size of about 0.2µm.

The invention also provides a *N.menimgitidis* vesicle composition, characterised in that the vesicles include (i) NspA protein, (ii) '287' protein and (iii) '741' protein.

### The bacterium

The bacterium from which OMVs are prepared may be Gram-positive, but it is preferably Gram-negative. The bacterium may be from genus *Moraxella, Shigella, Pseudomonas, Treporrema, Porphyromonas* or *Helicobacter* (see above for preferred species) but is preferably from the *Neisseria* genus. Preferred *Neisseria* species are *N.meningitidis* and *N.gonorrhoeae.* Within *N.meningitidis,* any of serogroups A, C, W135 and Y may be used, but it is preferred to prepare vesicles from serogroup B. Preferred strains within serogroup B are MC58, 2996, H4476 and 394/98.

To reduce pyrogenic activity, it is preferred that the bacterium should have low endotoxin (LPS) levels. Suitable mutant bacteria are known *e.g.* mutant *Neisseria* [23] and mutant *Helicobacter* [24]. Processes for preparing LPS-depleted outer membranes from Gram-negative bacteria are disclosed in reference 25

The bacterium may be a wild-type bacterium, or it may be a recombinant bacterium. Preferred recombinant bacteria over-express (relative to the corresponding wild-type strain) immunogens such as NspA, 287, 741, TbpA, TbpB, superoxide dismutase [6], *etc.* The bacterium may express more than one PorA class I outer membrane protein *e.g.* 2, 3, 4, 5 or 6 of PorA subtypes: P1.7,16; P1.5,2; P1.19,15; P1.5c,10; P1.12,13; and P1.7h,4 [*e.g*. refs. 26, 27].

The process of the invention will typically involve an initial step of culturing the bacteria, optionally followed by a step of concentrating the cultivated cells.

### Membrane disruption

Membrane disruption for vesicle formation is performed substantially in the absence of detergent.

In particular, membrane disruption may be performed substantially in the absence of a deoxycholate detergent, with other detergents optionally being present.

Membrane disruption may be performed substantially in the absence of ionic detergent, with non-ionic detergent optionally being present. Alternatively, it may be performed substantially in the absence of non-ionic detergent, with ionic detergent optionally being present. In some embodiments, neither ionic nor non-ionic detergent is present.

Steps after membrane disruption and vesicle formation may involve the use of detergent. Thus a process wherein membrane disruption occurs in the absence of detergent, but in which detergent is later added to the prepared vesicles, is encompassed within the invention.

The term "substantially in the absence" means that the detergent in question is present at a concentration of no more than 0.05% (*e.g.* ≤0.025%, ≤0.015%, ≤0.010%, <0.005%, ≤0.002%, ≤0.001% or even 0%) during membrane disruption. Thus processes where trace amounts of detergent are present during vesicle preparation are not excluded.

Membrane disruption in the absence of detergent may be performed on intact bacteria using physical techniques *e.g.* sonication, homogenisation, microfluidisation, cavitation, osmotic shock, grinding, French press, blending, *etc.*

### The vesicles

The processes of the invention produce outer membrane vesicles. OMVs are prepared from the outer membrane of cultured bacteria. They may be obtained from bacteria grown in broth or in solid medium culture, preferably by separating the bacterial cells from the culture medium (*e.g.* by filtration or by a low-speed centrifugation to pellet the cells), lysing the cells (without detergent), and separating an outer membrane fraction from cytoplasmic molecules (*e.g*. by filtration, by differential precipitation or aggregation of outer membranes and/or OMVs, by affinity separation methods using ligands that specifically recognize outer membrane molecules, or by a high-speed centrifugation that pellets outer membranes and/or OMVs).

OMVs can be distinguished from microvesicles (MVs [28]) and 'native OMVs' ('NOMVs' [66]), which are naturally-occurring membrane vesicles that form spontaneously during bacterial growth and are released into culture medium. MVs can be obtained by culturing *Neisseria* in broth culture medium, separating whole cells from the broth culture medium (*e.g.* by filtration or by low-speed centrifugation to pellet only the cells and not the smaller blebs) and then collecting the MVs that are present in the cell-depleted medium (*e.g.* by filtration, by differential precipitation or aggregation of MVs, by high-speed centrifugation to pellet the MVs). Strains for use in production of MVs can generally be selected on the basis of the amount of MVs produced in culture. References 29 and 30 describe *Neisseria* with high MV production.

### Retained bacterial immunogenic components

The substantial absence of detergent in processes of the invention results in vesicle preparations which retain immunogenic components of the bacterial surface which, using detergent-based prior art methods, would otherwise be lost or decreased. In *N.meningitidis,* three immunogens which are advantageously retained using the invention include, but are not limited to: (1) NspA; (2) protein '741'; and (3) protein '287'.

NspA (Neisserial surface protein A) is disclosed in references refs. 31 to 37 and as SEQ IDs 4008-4033 of reference 38. It is a candidate vaccine for the prevention of meningococcal disease. It is highly conserved between strains. Despite initial hope, however, it is now believed that NspA will not be an adequate protective antigen on its own and will need to be administered with additional antigens [*e.g.* ref. 36, and example 11 of ref. 38]. NspA has been found to be removed by prior art detergent-based preparation methods. According to the present invention, however, NspA can be retained in vesicles. Such NspA ^{+ve} vesicles are advantageous because a combination of two known potent immunogens (*i.e.* vesicles + NspA) is prepared in a single process, with each immunogen enhancing the efficacy of the other.

Protein '741' is disclosed as 'NMB1870' in reference 39 (GenBank: AAF42204, GI:7227128). It is also disclosed in references 40 and 41. It elicits strong bactericidal antibodies. It has been found that protein '741' is partially removed in vesicles prepared by prior art detergent-based methods. According to the present invention, however, '741' can be retained in vesicles. Such 741^{+ve} vesicles are advantageous because a combination of two known potent immunogens *(i.e.* vesicles + 741) is prepared in a single process, with each immunogen enhancing the efficacy of the other.

Protein '287' is disclosed as 'NMB2132' in reference 39 (GenBank: AAF42440, GI:7227388). It is also disclosed in references 40 and 42. It elicits strong bactericidal antibodies. Protein '287' is typically not present in vesicles prepared by prior art detergent-based methods and, to overcome its removal, it has previously been proposed that OMV preparations might be supplemented with 287 [43]. According to the present invention, however, '287' can be retained in vesicles. Such 287^{+ve} vesicles are advantageous because a combination of two known potent immunogens *(i.e.* vesicles + 287) is prepared in a single process, with each immunogen enhancing the efficacy of the other.

Preferred NspA (a) has at least a% sequence identity to amino acid sequence GI:1518522 and/or (b) comprises a fragment of at least x amino acids from amino acid sequence GI:1518522. Preferred '741' (a) has at least b% sequence identity to amino acid sequence GI:7227128 and/or (b) comprises a fragment of at least x amino acids from amino acid sequence GI:7227128. Preferred '287' (a) has at least *c*% sequence identity to amino acid sequence GI:7227388 and/or (b) comprises a fragment of at least *x* amino acids from amino acid sequence GI:7227388. The values of *a, b* and *c* are independent from each other, but each value is at least 70 (*e.g.* 75, 80, 85, 90, 95, 96, 97, 98, 99, 99.5 or 100). The values of *x*, *y* and *z* are independent from each other, but each value is at least 8 (*e.g.* 9, 10, 11, 12, 13, 14, 15, 16, 17, 18, 19, 20, 25, 30, 35, 40, 50, 60, 70, 80, 90, 100, 150, 200, 250 *etc.*). Fragments preferably comprise epitopes.

Preferred NspA, 287 and 741 proteins substantially retain the ability of the wild-type proteins (as found in intact bacteria) to elicit bactericidal antibodies in patients.

### Immunogenic pharmaceutical compositions

The process of the invention provides a vesicle preparation. For administration to a patient, the vesicles are preferably formulated as immunogenic compositions, and more preferably as compositions suitable for use as a vaccine in humans (*e.g.* children or adults). Vaccines of the invention may either be prophylactic (*i.e.* to prevent infection) or therapeutic (*i.e.* to treat disease after infection), but will typically be prophylactic.

The composition of the invention is preferably sterile.

The composition of the invention is preferably pyrogen-free.

The composition of the invention generally has a pH of between 6.0 and 7.0, more preferably to between 6.3 and 6.9 *e.g.* 6.6±0.2. The composition is preferably buffered at this pH.

Other components suitable for human administration are disclosed in reference 44.

The composition will generally comprise an adjuvant. Preferred adjuvants to enhance effectiveness of the composition include, but are not limited to: (A) MF59 (5% Squalene, 0.5% Tween 80, and 0.5% Span 85, formulated into submicron particles using a microfluidizer) [see Chapter 10 of ref. 45; see also ref. 46]; (B) microparticles *(i.e.* a particle of ~100nm to ~150µm in diameter, more preferably ~200nm to ~30µm in diameter, and most preferably ~500nm to ~10µm in diameter) formed from materials that are biodegradable and non-toxic (*e.g*. a poly(α-hydroxy acid), a polyhydroxybutyric acid, a polyorthoester, a polyanhydride, a polycaprolactone *etc.*), with poly(lactide-co-glycolide) being preferred, optionally being charged surface (*e.g.* by adding a cationic, anionic, or nonionic detergent such as SDS (negative) or CTAB (positive) [*e.g.* refs. 47 & 48]); (C) liposomes [see Chapters 13 and 14 of ref. 45]; (D) ISCOMs [see Chapter 23 of ref. 45], which may be devoid of additional detergent [49]; (E) SAF, containing 10% Squalane, 0.4% Tween 80, 5% pluronic-block polymer L121, and thr-MDP, either microfluidized into a submicron emulsion or vortexed to generate a larger particle size emulsion [see Chapter 12 of ref. 45]; (F) Ribi^{™} adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox^{™}); (G) saponin adjuvants, such as QuilA or QS21 [see Chapter 22 of ref. 45], also known as Stimulon^{™}; (H) chitosan [*e.g.* 50]; (1) complete Freund's adjuvant (CFA) and incomplete Freund's adjuvant (IFA); (J) cytokines, such as interleukins (*e.g.* IL-1, IL-2, IL-4, IL-5, IL-6, IL-7, IL-12, *etc.),* interferons (*e.g.* interferon-y), macrophage colony stimulating factor, tumor necrosis factor, *etc.* [see Chapters 27 & 28 of ref. 45]; (K) a saponin (*e.g.* QS21) + 3dMPL + IL-12 (optionally + a sterol) [51]; (L) monophosphoryl lipid A (MPL) or 3-O-deacylated MPL (3dMPL) [*e.g.* chapter 21 of ref. 45]; (M) combinations of 3dMPL with, for example, QS21 and/or oil-in-water emulsions [52]; (N) oligonucleotides comprising CpG motifs [53] *i.e.* containing at least one CG dinucleotide, with 5-methylcytosine optionally being used in place of cytosine; (O) a polyoxyethylene ether or a polyoxyethylene ester [54]; (P) a polyoxyethylene sorbitan ester surfactant in combination with an octoxynol [55] or a polyoxyethylene alkyl ether or ester surfactant in combination with at least one additional non-ionic surfactant such as an octoxynol [56]; (Q) an immunostimulatory oligonucleotide (*e.g.* a CpG oligonucleotide) and a saponin [57]; (R) an immunostimulant and a particle of metal salt [58]; (S) a saponin and an oil-in-water emulsion [59]; (T) *E.coli* heat-labile enterotoxin ("LT"), or detoxified mutants thereof, such as the K63 or R72 mutants [*e.g.* Chapter 5 of ref. 60]; (U) cholera toxin ("CT"), or detoxified mutants thereof [*e.g.* Chapter 5 of ref. 60]; (V) double-stranded RNA; (W) aluminium salts, such as aluminium hydroxides (including oxyhydroxides), aluminium phosphates (including hydroxyphosphates), aluminium sulfate, *etc* [Chapters 8 & 9 in ref. 61]; (X) monophosphoryl lipid A mimics, such as aminoalkyl glucosaminide phosphate derivatives *e.g.* RC-529 [62]; (Y) polyphosphazene (PCPP); or (Z) a bioadhesive [63] such as esterified hyaluronic acid microspheres [64] or a mucoadhesive selected from the group consisting of cross-linked derivatives of poly(acrylic acid), polyvinyl alcohol, polyvinyl pyrollidone, polysaccharides and carboxymethylcellulose. Other substances that act as immunostimulating agents to enhance the effectiveness of the composition [*e.g.* see Chapter 7 of ref. 45] may also be used. Aluminium salts (especially aluminium phosphates and/or hydroxides) are preferred adjuvants for parenteral immunisation. Mutant toxins are preferred mucosal adjuvants.

The vesicles in the compositions of the invention will be present in 'immunologically effective amounts' i.e. the administration of that amount to an individual, either in a single dose or as part of a series, is effective for treatment or prevention of disease. This amount varies depending upon the health and physical condition of the individual to be treated, age, the taxonomic group of individual to be treated (*e.g.* non-human primate, primate, *etc.),* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Dosage treatment may be a single dose schedule or a multiple dose schedule (*e.g.* including booster doses). The vaccine may be administered in conjunction with other immunoregulatory agents.

Typically, the compositions of the invention are prepared as injectables. Direct delivery of the compositions will generally be parenteral (*e.g.* by injection, either subcutaneously, intraperitoneally, intravenously or intramuscularly or delivered to the interstitial space of a tissue) or mucosal (*e.g*. oral or intranasal [65,66]). The compositions can also be administered into a lesion.

Once formulated, the compositions of the invention can be administered directly to the subject. The subjects to be treated can be animals; in particular, human subjects can be treated. The vaccines are particularly useful for vaccinating children and teenagers.

The composition may comprise vesicles from more than one serosubtype of *N.meningitidis* [28]. Similarly, the composition may comprise more than one type of vesicle *e.g.* both MVs and OMVs

As well as vesicles, the composition of the invention may comprise further antigens. For example, the composition may comprise one or more of the following further antigens:
- antigens from *Helicobacter pylori* such as CagA [67 to 70], VacA [71, 72], NAP [73, 74, 75], HopX [*e.g.* 76], HopY [*e.g.* 76] and/or urease.
- a saccharide antigen from *N.meningitidis* serogroup A, C, W135 and/or Y, such as the oligosaccharide disclosed in ref. 77 from serogroup C [see also ref. 78] or the oligosaccharides of ref. 79.
- a saccharide antigen from *Streptococcus pneumoniae* [*e.g.* 80, 81, 82].
- an antigen from hepatitis A virus, such as inactivated virus [*e.g.* 83, 84].
- an antigen from hepatitis B virus, such as the surface and/or core antigens [*e.g.* 84, 85].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 [*e.g.* refs. 86 & 87].
- a diphtheria antigen, such as a diphtheria toxoid [*e.g.* chapter 3 of ref. 88] *e.g.* the CRM₁₉₇ mutant [*e.g.* 89].
- a tetanus antigen, such as a tetanus toxoid [*e.g.* chapter 4 of ref. 108].
- a saccharide antigen from *Haemophilus influenzae B* [*e.g.* 78].
- an antigen from hepatitis C virus [*e.g.* 90].
- an antigen from *N.gonorrhoeae* [*e.g.* 91, 92, 93, 94].
- an antigen from *Chlamydia pneumoniae* [*e.g.* refs. 95 to 101].
- an antigen from *Chlamydia trachomatis* [*e.g.* 102].
- an antigen from *Porphyromonas gingivalis* [*e.g.* 103].
- polio antigen(s) [*e.g.* 104, 105] such as OPV or, preferably, IPV.
- rabies antigen(s) [*e.g.* 106] such as lyophilised inactivated virus *[e.g.* 107, RabAvert^{™}].
- measles, mumps and/or rubella antigens *[e.g.* chapters 9, 10 & 11 of ref. 108].
- influenza antigen(s) [*e.g.* chapter 19 of ref. 108], such as the haemagglutinin and/or neuraminidase surface proteins.
- an antigen from *Moraxella catarrhalis* [*e.g.* 109].
- an protein antigen from *Streptococcus agalactiae* (group B streptococcus) *[e.g.* 110, 111].
- a saccharide antigen from *Streptococcus agalactiae* (group B streptococcus).
- an antigen from *Streptococcus pyogenes* (group A streptococcus) *[e.g.* 111, 112, 113].
- an antigen from *Staphylococcus aureus* [*e.g.* 114].
- an antigen from *Bacillus anthracis* [*e.g* 115, 116, 117].
- an antigen from a virus in the flaviviridae family (genus flavivirus), such as from yellow fever virus, Japanese encephalitis virus, four serotypes of Dengue viruses, tick-borne encephalitis virus, West Nile virus.
- a pestivirus antigen, such as from classical porcine fever virus, bovine viral diarrhoea virus, and/or border disease virus.
- a parvovirus antigen *e.g.* from parvovirus B19.
- a prion protein (*e.g*. the CJD prion protein)
- an amyloid protein, such as a beta peptide [118]
- a cancer antigen, such as those listed in Table 1 of ref. 119 or in tables 3 & 4 of ref. 120.

The composition may comprise one or more of these further antigens.

Toxic protein antigens may be detoxified where necessary (*e.g.* detoxification of pertussis toxin by chemical and/or genetic means [87]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

Saccharide antigens are preferably in the form of conjugates. Carrier proteins for the conjugates include the *N.meningitidis* outer membrane protein [121], synthetic peptides [122,123], heat shock proteins [124,125], pertussis proteins [126,127], protein D from *H.influenzae* [128], cytokines [129], lymphokines [129], hormones [129], growth factors [129], toxin A or B from *C.difficile* [130], iron-uptake proteins [131], *etc.* A preferred carrier protein is the CRM197 diphtheria toxoid [132].

*N.meningitidis* serogroup B antigens may also be added to the OMV compositions. In particular, a protein antigen such as disclosed in refs. 133 to 139 may be added.

Antigens in the composition will typically be present at a concentration of at least 1 µg/ml each. In general, the concentration of any given antigen will be sufficient to elicit an immune response against that antigen.

As an alternative to using protein antigens in the composition of the invention, nucleic acid encoding the antigen may be used. Protein components of the compositions of the invention may thus be replaced by nucleic acid (preferably DNA *e.g.* in the form of a plasmid) that encodes the protein.

### Methods of treating patients

The invention provides vesicles of the invention for use as medicaments.

Vesicles of the invention can be used in a method of raising an immune response in a patient, comprising administering to a patient a composition of the invention. The immune response is preferably protective against meningococcal disease, and may comprise a humoral immune response and/or a cellular immune response. The patient is preferably a child.

The method may raise a booster response, in a patient that has already been primed against *N.meningitidis.* Subcutaneous and intranasal prirne/boost regimes for OMVs are disclosed in ref. 65.

The invention also provides the use of a vesicle of the invention in the manufacture of a medicament for raising an immune response in an patient. The medicament is preferably an immunogenic composition (*e.g.* a vaccine). The medicament is preferably for the prevention and/or treatment of a disease caused by a *Neisseria* (*e.g.* meningitis, septicaemia, gonorrhoea *etc.),*

These methods and uses may involve administration of vesicles from more than one serosubtype of *N meningitidis* [*e.g.* ref. 28].

### OMV formulation

The invention provides a composition comprising meningococcal outer membrane vesicles, an aluminium hydroxide adjuvant, a histidine buffer and sodium chloride, wherein: (a) the concentration of sodium chloride is greater than 7.5 mg/ml; and/or (b) the concentration of OMVs is less than 100 µg/ml.

The concentration of sodium chloride is preferably greater than 8 mg/ml, and is more preferably about 9 mg/ml.

The concentration of OMVs is preferably less than 75 mg/ml *e.g.* about 50 mg/ml.

The histidine buffer is preferably between pH 6.3 and pH 6.7 *e.g.* pH 6.5.

The adjuvant may be used at about 3.3 mg/ml (expressed as Al³⁺ concentration).

### Definitions

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of reference 140. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is well known and is disclosed in reference 141.

The term "comprising" means "including" as well as "consisting" *e.g.* a composition "comprising" X may consist exclusively of X or may include something additional *e.g.* X + Y.

The term "about" in relation to a numerical value *x* means, for example, x±10%.

The word "substantially" does not exclude "completely" *e.g.* a composition which is "substantially free" from Y may be completely free from Y. Where necessary, the word "substantially" may be omitted from the definition of the invention.

### BRIEF DESCRIPTION OF DRAWINGS

Figures 1 and 2 show the presence/absence of (1) protein '287' and (2) protein '741' in bacteria ('TOT') and outer membrane vesicles ('OMV') prepared from strains MC58, H4476 and 394/98 of *N.meningitidis.* The arrow shows the position of '287' in Figure 1 and `741' in Figure 2.

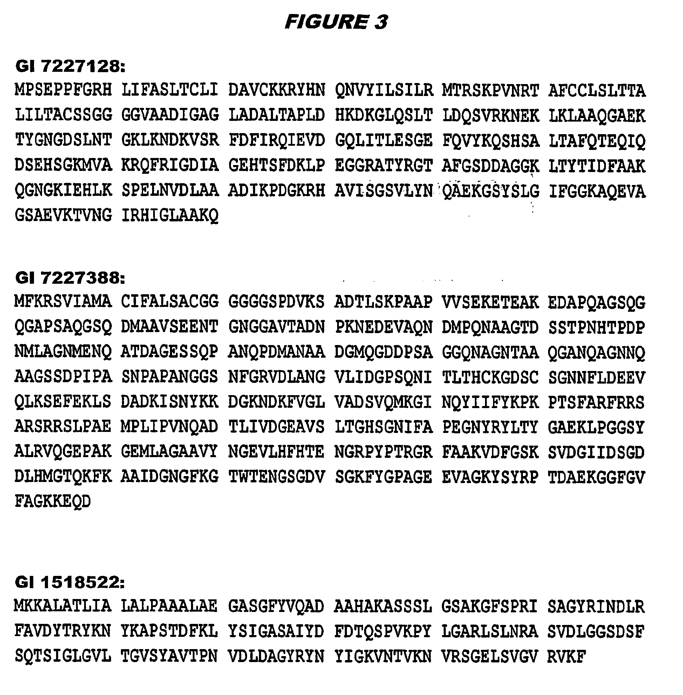

Figure 3 shows the amino acid sequences of GenBank entries GI:7227128, GI:7227388 and GI:1518522 as of 29th August 2002.

### MODES FOR CARRYING OUT THE INVENTION

### OMV preparation

OMVs were prepared either by the prior art 'Norwegian' methods (strains H4476 and 394/98) or by the following process (strain MC58):
- Bacteria from 2-5 plates were harvested into 10 ml of 10mM Tris-HCl buffer (pH 8.0) and heat-killed at 56°C for 45min. The samples were then sonicated on ice (duty cycle 50 for 10 minutes with the tip at 6/7) to disrupt membranes.
- Cellular debris was removed by centrifugation at 5000g for 30 minutes at 4°C, or 10000g for 10 minutes.
- The supernatant was re-centrifuged at 50000g for 75 minutes at 4°C
- The pellet was resuspended in 7 ml of 2% N-lauroyl sarcosinate (Sarkosyl) in 10 mM Tris-HCl (pH 8.0) for 20 minutes at room temperature to solubilise the cytoplasmic membranes.
- The sample was centrifuged at 10000g for 10 minutes to remove particulates and the supernatant was centrifuged at 75000g for 75 minutes at 4°C. The sample was washed in 10mM Tris-HCl (pH 8.0) and centrifuged at 75000g for 75 minutes.
- The pellet was resuspended in 10 mM Tris-HCl (pH 8.0) or distilled water.

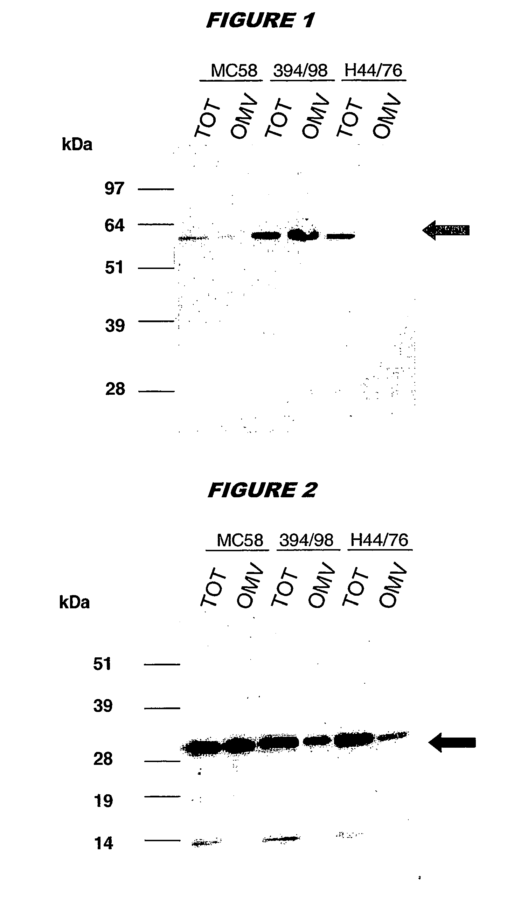

The bacteria and the OMV preparations were tested by Western blot for the presence of NspA, 287 and 741 (Figures 1 & 2) and results are summarised in the following table:

| **Strain** | **Detergent** | **NspA** | | **287** | | **741** | |
|---|---|---|---|---|---|---|---|
| | | **Bacteria** | **OMV** | **Bacteria** | **OMV** | **Bacteria** | **OMV** |
| MC58 | - | +++ | +++ | +++ | +++ | +++ | +++ |
| H44/76 | + | +++ | - ⁽²⁰⁾ | +++ | - | +++ | + |
| 394/98 | + | +++ | n.d. | +++ | ++ | +++ | + |

In contrast to the prior art detergent-based methods, therefore, the absence of detergent results in NspA being retained in OMVs and avoids loss of 287 & 741.

### Formulation of OMVs prepared from New Zealand strain ofMenB

OMVs were prepared from serogroup B strain 394/98 of *N.meningitidis.* These were formulated in two different ways, with components having the following concentrations:

| | **Formulation 'A'** | **Formulation 'B'** |
|---|---|---|
| **OMVs** | 50 µg/ml | 50 µg/ml |
| **Aluminium hydroxide adjuvant** | 3.3 µg.ml | 3.3 µg.ml |
| **Sucrose** | 3% | - |
| **Histidine buffer, pH 6.5** | - | 5 mM |
| **Sodium chloride** | - | 9 mg/ml |

Formulation 'B' was found to be immunologically superior to formulation 'A'. Formulation 'B' differs from that disclosed in reference 142 by having half the OMV concentration a higher NaCl concentration, and a slightly different pH.

It will be understood that the invention has been described by way of example only and modifications may be made whilst remaining within the scope of the invention.

### REFERENCES

[1] Bjune et al. (1991) Lancet 338(8775):1093-1096.
[2] de Kleijn et al. (2001) Vaccine 20:352-358.
[3] US patents 5,597,572 & 5,747,653; see also European patent 0301992.
[4] European patent 0449958 (granted from WO0/06696).
[5] US patent 5,705,161; see also WO4/08021.
[6] International patent application WO00/25811.
[7] International patent application WO01/52885.
[8] International patent application WO98/56901.
[9] International patent application WO01/91788.
[10] Parmar et al. (1997) Vaccine 15:1641-1651.
[11] International patent application WO99/59625.
[12] International patent application WO00/50074.
[13] US patents 5,552,146, 5,981,213 & 5,993,826; see also WO93/03761.
[14] Zhou et al. (1998) FEMS Microbiol Lett 163:223-228.
[15] Kadurugamuwa & Beveridge (1999) Microbiology 145:2051-2060.
[16] International patent application WO97/05899.
[17] Kesavalu et al. (1992) Infect. Immun. 60:1455-1464.
[18] Blanco et al. (1999) J Immunol 163:2741-2746.
[19] International patent application WO01/09350.
[20] International patent application WO02/09746.
[21] Keenan et al. (1998) FEMS Microbiol Lett 161:21-27.
[22] European patent 0011243.
[23] International patent application WO99/10497.
[24] International patent application WO02/07763.
[25] European patent 0624376.
[26] Claassen et al. (1996) Vaccine 14:1001-1008.
[27] Peeters et al. (1996) Vaccine 14:1009-1015.
[28] International patent application WO02/09643.
[29] US patent 6,180,111.
[30] International patent application WO01/34642.
[31] Martin et al. (1997) J.Exp. Med. 185:1173-1183.
[32] Plante et al. (1999) Infect. Immun. 67:2855-2861.
[33] Cadieux et al. (1999) Infect. Immun. 67:4955-4959.
[34] Moe et al. (1999) Infect. Immun. 67:5664-5675.
[35] Martin et al. (2000) J. Biotechnol. 83:27-31.
[36] Moe et al. (2001) Infect. Immun. 69:3762-3771.
[37] International patent application WO96/29412.
[38] International patent application WO00/71725.
[39] Tettelin et al. (2000) Science 287:1809-1815.
[40] International patent application WO9/57280.
[41] International patent application WO3/020756 (SEQ IDs 1-22 in particular).
[42] International patent application WO00/66741.
[43] International patent application WO01/52885.
[44] Gennaro (2000) Remington: The Science and Practice of Pharmacy. 20th edition, ISBN: 0683306472.
[45] Vaccine design: the subunit and adjuvant approach, eds. Powell & Newman, Plenum Press 1995 (ISBN 0-306-44867-X).
[46] WO90/14837.
[47] WO02/26212.
[48] WO98/33487.
[49] WOOO/07621.
[50] WO9/27960.
[51] WO98/57659.
[52] European patent applications 0835318, 0735898 and 0761231.
[53] Krieg (2000) Vaccine 19:618-622; Krieg (2001) Curr opin Mol Ther 2001 3:15-24; WO96/02555, WO98/16247, WO98/18810, WO98/40100 WO98/55495, WO98/37919 and WO98/52581 *etc*.
[54] WO99/52549.
[55] WO01/2120
[56] WO01/21152.
[57] WO00/6280
[58] WO00/23105.
[59] WO9/11241.
[60] Del Giudice et al. (1998) Molecular Aspects of Medicine, vol. 19, number 1.
[61] Vaccine Design... (1995) eds. Powell & Newman. ISBN: 030644867X. Plenum.
[62] Johnson et al. (1999) Bioorg Med Chem Lett 9:2273-2278.
[63] International patent application WO00/50078.
[64] Singh et al. (2001) J. Cont. Rele. 70:267-276.
[65] Bakke et al. (2001) Infect. Immun. 69:5010-5015.
[66] Katial et al. (2002) Infect. Immun. 70:702-707.
[67] Covacci & Rappuoli (2000) J. Exp. Med. 19:587-592.
[68] WO3/18150.
[69] Coacci et al. (1993) Proc. Natl. Acad. Sci. USA 90: 5791-5795.
[70] Tummuru et al. (1994) Infect. Immun. 61:1799-1809.
[71] Marchetti et al. (1998) Vaccine 16:33-37.
[72] Telford et al. (1994) J. Exp. Med. 179:1653-1658.
[73] Evans et al. (1995) Gene 153:123-127.
[74] WO96/01272 & WO96/01273, especially SEQ ID NO:6.
[75] WO97/25429.
[76] WO98/04702.
[77] Costantino et al. (1992) Vaccine 10:691-698.
[78] Costantino et al. (1999) Vaccine 17:1251-1263.
[79] International patent application WO03/007985.
[80] Watson (2000) Pediatr Infect Dis J 19:331-332.
[81] Rubin (2000) Pediatr Clin North Am 47:269-285, v.
[82] Jedrzejas (2001) Microbiol Mol Biol Rev 65:187-207.
[83] Bell (2000) Pediatr Infect Dis J 19:1187-1188.
[84] Iwarson (1995) APMIS 103:321-326.
[85] Gerlich et al. (1990) Vaccine 8 Suppl:S63-68 & 79-80.
[86] Gustafsson et al. (1996) N. Engl. J. Med. 334:349-355.
[87] Rappuoli et al. (1991) TIBTECH 9:232-238.
[88] Vaccines (1988) eds. Plotkin & Mortimer. ISBN 0-7216-1946-0.
[89] Del Guidice et al. (1998) Molecular Aspects of Medicine 19:1-70.
[90] Hsu et al. (1999) Clin Liver Dis 3:901-915.
[91] International patent application WO99/24578.
[92] International patent application WO99/36544.
[93] International patent application WO99/57280.
[94] International patent application WO02/07924
[95] International patent application WO02/02606.
[96] Kalman et al. (1999) Nature Genetics 21:385-389.
[97] Read et al. (2000) Nucleic Acids Res 28:1397-406*.*
[98] Shirai et al. (2000) J. Infect. Dis. 181(Suppl 3):S524-S527.
[99] International patent application WO99/27105.
[100] International patent application WO00/27994.
[101] International patent application WO00/37494.
[102] International patent application WO99/28475.
[103] Ross et al. (2001) Vaccine 19:4135-4142.
[104] Sutter et al. (2000) Pediatr Clin North Am 47:287-308.
[105] Zimmerman & Spann (1999) Am Fam Physician 59:113-118, 125-126.
[106] Dreesen (1997) Vaccine 15 Suppl:S2-6.
[107] MMWR Morb Mortal Wkly Rep 1998 Jan 16;47(1):12, 19.
[108] Vaccines (1988) eds. Plotkin & Mortimer. ISBN 0-7216-1946-0.
[109] McMichael (2000) Vaccine 19 Suppl 1:S101-107.
[110] Schuchat (1999) Lancet 353(9146):51-6.
[111] International patent application WO02/34771.
[112] Dale (1999) Infect Dis Clin North Am 13:227-43, viii.
[113] Ferretti et al. (2001) PNAS USA 98: 4658-4663.
[114] Kuroda et al. (2001) Lancet 357(9264):1225-1240; see also pages 1218-1219.
[115] J Toxicol Clin Toxicol (2001) 39:85-100.
[116] Demicheli et al. (1998) Vaccine 16:880-884.
[117] Stepanov et al. (1996) J Biotechnol 44:155-160.
[118] Ingram (2001) Trends Neurosci 24:305-307.
[119] Rosenberg (2001) Nature 411:380-384.
[120] Moingeon (2001) Vaccine 19:1305-1326.
[121] EP-A-0372501
[122] EP-A-0378881
[123] EP-A-0427347
[124] WO93/17712
[125] WO94/03208
[126] WO8/58668
[127] EP-A-0471177
[128] WO00/56360
[129] WO1/01146
[130] WO00/61761
[131] WO01/723
[132] Research Disclosure, 453077 (Jan 2002)
[133] WO99/24578.
[134] WO9/36544.
[135] WO99/57280.
[136] WO00/22430.
[137] Tettelin et al. (2000) Science 287:1809-1815.
[138] WO96/29412.
[139] Pizza et al. (2000) Science 287:1816-1820.
[140] Current Protocols in Molecular Biology (F.M. Ausubel et al., eds., 1987) Supplement 30.
[141] Smith and Waterman, Adv. Appl. Math. (1981) 2: 482-489.
[142] WO03/009869.

## Claims

1. A process for the manufacture of an outer membrane vesicle preparation from a bacterium, wherein the bacterial membrane is disrupted substantially in the absence of deoxycholate detergent and the bacterium over-expresses TbpA.

2. The process of claim 1, wherein the bacterial membrane is disrupted substantially in the absence of any detergent.

3. The process of claim 1 or claim 2, comprising the following basic steps: (a) treating bacterial cells in the substantial absence of detergent; (b) centrifuging the composition from step (a) to separate the outer membrane vesicles from treated cells and cell debris, and collecting the supernatant; (c) performing a high speed centrifugation of the supernatant from step (b) and collecting the outer membrane vesicles in a pellet; (d) re-dispersing the pellet from step (c) in a buffer; (e) performing a second high speed centrifugation in accordance with step (c), collecting the outer membrane vesicles in a pellet; (f) re-dispersing the pellet from step (e) in an aqueous medium.

4. The process of claim 3, further comprising the following steps: (g) performing sterile filtration through at least two filters of decreasing pore size of the re-dispersed composition from step (f); and (h) optionally including the composition from step (g) in a pharmaceutically acceptable carrier and/or adjuvant composition.

5. The process of claim 3 or claim 4, wherein step (b) comprises centrifugation at around 5000 - 10000 g for up to 1 hour, and steps (c) and (e) comprise centrifugation at around 35000 - 100000 g for up to 2 hours.

6. The process of any preceding claim, wherein membrane disruption is by sonication, homogenisation, microfluidisation, cavitation, osmotic shock, grinding, French press, blending, or any other physical technique.

7. The process of any preceding claim, wherein the buffer used in step (d) and/or in step (f) is a Tris buffer, a phosphate buffer, or a histidine buffer.

8. The process of any one of claims 5 to 7, wherein step (g) ends with a filter of pore-size of about 0.2µm.

9. The process of any preceding claim, wherein the bacterium from which OMVs are prepared is from genus *Moraxella, Shigella, Pseudonconas, Treponema, Porphyromonas, Helicobacter* or *Neisseria.*

10. The process of claim 9, wherein the bacterium is *N.meningitidis* or *N.gonorrhoeae.*

11. The process of claim 10, wherein the *N.meningitidis* is from serogroup B.

12. The process of claim 11, wherein the bacterium is *Neisseria meningitidis* serogroup B strain H4476.

13. The process of any preceding claim, further comprising the step of formulating an immunologically effective amount of the OMVs as an immunogenic composition

14. An OMV composition obtainable by the process of any preceding claim.

15. A *Neisseria meningitidis* vesicle composition, **characterised in that** the vesicles include (i) NspA protein, (ii) '287' protein and (iii) '741' protein.

16. The composition of claim 14 or claim 15, wherein the composition is sterile and/or pyrogen-free and/or buffered at a pH of between 6.0 and 7.0.

17. The composition of any one of claims 14 to 16 for use as a medicament.

18. The use of a composition of any one of claims 14 to 16 in the manufacture of a medicament for raising an immune response in an patient.

## Patentansprüche

1. Verfahren zur Herstellung eines äußere Membranvesikel-Präparats von einem Bakterium, wobei die Bakterienmembran im Wesentlichen in Abwesenheit von Desoxycholat-Detergens zerstört wird und das Bakterium TbpA überexprimiert.

2. Verfahren gemäß Anspruch 1, wobei die Bakterienmembran im Wesentlichen in der Abwesenheit eines beliebigen Detergens zerstört wird.

3. Verfahren gemäß Anspruch 1 oder 2, das die folgenden grundlegenden Schritte umfasst: (a) das Behandeln von Bakterienzellen im Wesentlichen in der Abwesenheit von Detergens; (b) das Zentrifugieren der Zusammensetzung von Schritt (a), um die äußere Membranvesikel von behandelten Zellen und Zelltrümmern zu trennen, und das Sammeln des Überstandes; (c) das Durchführen einer Hochgeschwindigkeitszentrifugation des Überstandes von Schritt (b) und das Sammeln der äußeren Membranvesikel in einem Niederschlag; (d) das Wiederdispergieren des Niederschlags aus Schritt (c) in einem Puffer; (e) das Durchführen einer zweiten Hochgeschwindigkeitszentrifugation in Übereinstimmung mit Schritt (c), das Sammeln der äußeren Membranvesikel in einem Niederschlag; und (f) das Wiederdispergieren des Niederschlags von Schritt (e) in einem wässrigen Medium.

4. Verfahren gemäß Anspruch 3, das ferner die folgenden Schritte umfasst: (g) das Durchführen einer Sterilfiltration der wiederdispergierten Zusammensetzung von Schritt (f) durch mindestens zwei Filter mit abnehmender Porengröße; und (h), gegebenenfalls, das Aufnehmen der Zusammensetzung von Schritt (g) in einen/eine pharmazeutisch-verträgliche(n) Träger und/oder Hilfszusammensetzung.

5. Verfahren gemäß Anspruch 3 oder 4, wobei Schritt (b) eine Zentrifugation bei etwa 5000 bis 10000 g für bis zu 1 Stunde umfasst, und die Schritte (c) und (e) eine Zentrifugation bei etwa 35000 bis 100000 g für bis zu 2 Stunden umfassen.

6. Verfahren gemäß einem der vorhergehenden Ansprüche, wobei die Membranzerstörung durch Ultraschall, Homogenisieren, Mikrofluidisieren, Kavitation, osmotischen Schock, Zerreiben, *French press,* Mischen oder irgendeine andere physikalische Technik geschieht.

7. Verfahren gemäß einem der vorhergehenden Ansprüche, wobei der Puffer, der in Schritt (d) und/oder in Schritt (f) verwendet wird, ein Tris-Puffer, ein Phosphatpuffer, oder ein Histidin-Puffer ist.

8. Verfahren gemäß einem der Ansprüche 5 bis 7, wobei Schritt (g) mit einem Filter der Porengröße von etwa 0.2 µm endet.

9. Verfahren gemäß einem der vorhergehenden Ansprüche, wobei das Bakterium, von dem die OMVs hergestellt werden, von der Gattung *Moraxella, Shigella, Pseudomonas, Treponema, Porphyromonas, Heliobacter* oder *Neisseria* ist.

10. Verfahren gemäß Anspruch 9, wobei das Bakterium *N. meningitidis* oder *N. gonorrhoeae* ist.

11. Verfahren gemäß Anspruch 10, wobei *N. meningitidis* aus der Serogruppe B ist.

12. Verfahren gemäß Anspruch 11, wobei das Bakterium *Neisseria meningitidis* Serogruppe B Stamm H4476 ist.

13. Verfahren gemäß einem der vorhergehenden Ansprüche, das ferner den Schritt des Formulierens einer immunologisch wirksamen Menge der OMVs als eine immunogene Zusammensetzung umfasst.

14. OMV-Zusammensetzung, erhältlich durch das Verfahren gemäß einem der vorhergehenden Ansprüche.

15. *Neisseria* meningitidis-Vesikelzusammensetzung, **dadurch gekennzeichnet, dass** die Vesikel (i) NspA-Protein, (ii) '287'-Protein und (iii) '741'-Protein beinhalten.

16. Zusammensetzung gemäß Anspruch 14 oder 15, wobei die Zusammensetzung steril und/oder pyrogenfrei und/oder bei einem pH-Wert zwischen 6.0 und 7.0 gepuffert ist.

17. Zusammensetzung gemäß einem der Ansprüche 14 bis 16 zur Verwendung als ein Arzneimittel.

18. Verwendung einer Zusammensetzung gemäß einem der Ansprüche 14 bis 16 für die Herstellung eines Arzneimittels zum Hervorrufen einer Immunantwort in einem Patienten.

## Revendications

1. Procédé pour la fabrication d'une préparation de vésicules de membrane extérieure à partir d'une bactérie, dans lequel la membrane bactérienne est rompue pratiquement en l'absence de détergent à base de désoxycholate et la bactérie sur-exprime TbpA.

2. Procédé selon la revendication 1, dans lequel la membrane bactérienne est rompue pratiquement en l'absence d'un détergent quelconque.

3. Procédé selon la revendication 1 ou la revendication 2, comprenant les étapes basiques suivantes : (a) traitement de cellules bactériennes pratiquement en l'absence de détergent ; (b) centrifugation de la composition obtenue dans l'étape (a) pour séparer les vésicules de membrane extérieure d'avec les cellules traitées et les débris cellulaires, et collecte du surnageant ; (c) mise en oeuvre d'une centrifugation à grande vitesse du surnageant obtenu dans l'étape (b) et collecte des vésicules de membrane extérieure dans un culot ; (d) re-dispersion du culot obtenu dans l'étape (c) dans un tampon, (e) mise en oeuvre d'une deuxième centrifugation à grande vitesse conformément à l'étape (c), collecte des vésicules de membrane extérieure dans un culot ; (f) re-dispersion du culot obtenu dans l'étape (e) dans un milieu aqueux.

4. Procédé selon la revendication 3, comprenant en outre les étapes suivantes : (g) mise en oeuvre d'une filtration stérile sur au moins deux filtres ayant des tailles de pores décroissantes de la composition re-dispersée obtenue dans l'étape (f) ; et (h) éventuellement incorporation de la composition obtenue dans l'étape (g) dans un véhicule acceptable en pharmacie et/ou une composition adjuvante.

5. Procédé selon la revendication 3 ou la revendication 4, dans lequel l'étape (b) comprend une centrifugation à environ 5 000 à 10 000 g pendant jusqu'à 1 heure, et les étapes (c) et (e) comprennent une centrifugation à environ 35 000 à 100 000 g pendant jusqu'à 2 heures.

6. Procédé selon l'une quelconque des revendications précédentes, dans lequel la rupture de membrane est effectuée par sonification, homogénéisation, microfluidisation, cavitation, choc osmotique, broyage, presse French, mélange, ou n'importe quelle autre technique physique.

7. Procédé selon l'une quelconque des revendications précédentes, dans lequel le tampon utilisé dans l'étape (d) et/ou dans l'étape (f) est un tampon Tris, un tampon phosphate, ou un tampon histidine.

8. Procédé selon l'une quelconque des revendications 5 à 7, dans lequel l'étape (g) se termine par un filtre ayant une taille de pores d'environ 0,2 µm.

9. Procédé selon l'une quelconque des revendications précédentes, la bactérie à partir de laquelle les OMV sont préparées appartient au genre *Moraxella, Shigella, Pseudomonas, Treponema, Porphyromonas, Helicobacter* ou *Neisseria.*

10. Procédé selon la revendication 9, dans lequel la bactérie est *N. meningitidis* ou *N. gonorrhoeae.*

11. Procédé selon la revendication 10, dans lequel *N. meningitidis* appartient au sérogroupe B.

12. Procédé selon la revendication 11, dans lequel la bactérie est la souche H4476 de *Neisseria meningitidis* sérogroupe B.

13. Procédé selon l'une quelconque des revendications précédentes, comprenant en outre l'étape de formulation d'une quantité immunologique efficace des OMV sous la forme d'une composition immunogène.

14. Composition d'OMV pouvant être obtenue par le procédé de l'une quelconque des revendications précédentes.

15. Composition de vésicules de *Neisseria meningitidis,* **caractérisée en ce que** les vésicules comprennent (i) la protéine NapA, (ii) la protéine '287' et (iii) la protéine '741'.

16. Composition selon la revendication 14 ou la revendication 15, dans laquelle la composition est stérile et/ou apyrogène et/ou tamponnée à un pH compris entre 6,0 et 7,0.

17. Composition selon l'une quelconque des revendications 14 à 16, pour une utilisation en tant que médicament.

18. Utilisation d'une composition selon l'une quelconque des revendications 14 à 16, dans la fabrication d'un médicament pour diriger une réponse immunitaire chez un patient.
